# EUROPEAN PATENT SPECIFICATION

(11) **EP 2 032 126 B1**
(45) Date of publication and mention of the grant of the patent: **27.07.2011**
(21) Application number: 07764889.7
(22) Date of filing: 27.06.2007
(51) Int. Cl.: A61K 9/36, A61K 9/32, A61K 31/4439

(54) **RABEPRAZOLE FORMULATION**
RABEPRAZOL-FORMULIERUNG
PRÉPARATION PHARMACEUTIQUE CONTENANT DU RABÉPRAZOLE

(30) Priority: 29.06.2006 EP 06013440
(43) Date of publication of application: 11.03.2009
(73) Proprietor: LEK Pharmaceuticals d.d., 1526 Ljubljana (SI)
(72) Inventor: JURECIC, Rok, 1000 Ljubljana (SI); JAKLIC, Miha Tomaz, 1262 Dol Pri Ljubljani (SI)
(74) Representative: Oser, Andreas
(86) International application number: PCT/EP2007/005691
(87) International publication number: WO 2008/000463

(56) References cited:
- EP-A- 1 004 305
- WO-A-2004/075881
- WO-A-2005/027876
- US-A- 4 853 230
- US-A1- 2005 042 277
- JANSSEN-CILAG B.V.: "Deel IB1 Pariet, version August 1, 2005" INTERNET ARTICLE, [Online] August 2005 (2005-08), XP002408249 Retrieved from the Internet: URL:http://www.cbg-meb.nl/nl/prodinfo/inde x.htm> [retrieved on 2006-11-21]

## Description

### Field of the invention

The present invention belongs to the field of pharmaceutical technology and relates to an improved pharmaceutical composition comprising rabeprazole and/or a pharmaceutically acceptable salt thereof such as rabeprazole sodium.

### Background of the invention

Rabeprazole has been disclosed in European patent EP 268956 presenting the proton pump inhibition activity thereof, a synthesis and a formulation. Rabeprazole may be used for treatment or prevention of peptic ulcers.

Stability of rabeprazole sodium *per se* is poor. In particular it is rapidly decomposed and coloured under moist conditions or in an acidic to neutral aqueous solution. Therefore when rabeprazole sodium is to be formulated into a preparation for oral administration, it should be enteric coated to prevent the decomposition thereof with the gastric acid. However an enteric coating is an acidic material which is insoluble in water in acidic conditions and soluble in water in neutral to alkaline conditions. Thus the coating of the core comprising an acid-unstable compound might generally cause the decomposition of said acid-unstable compound. Such decomposition occurs even during the enteric coating stage by a common method, for example, by using a fluidized bed coater, which results in the colouration of the surface of the core. Furthermore the storage stability of the coated core as well as the stability in an acidic solution of the same might thereby be lowered.
In order to avoid these difficulties, Japanese Patents Laid-Open No. 258316/1987 and No. 258320/1987 disclose a method comprising intermediately coating the core containing an acid-unstable compound with a material soluble or decomposable in water and then further coating the same with an enteric coating. However these methods cannot sufficiently stabilize an acid-unstable compound and therefore further improvement is required.

A European patent application EP 1004305 discloses a composition of rabeprazole sodium with a specially selected excipients, such as potassium carbonate, sodium hydroxide, potassium hydroxide, and other fillers, such as aminoalkyl methacrylate copolymer E. Those fillers are found to be compatible with rabeprazole sodium but for example sodium hydroxide is a very hygroscopic compound and therefore could cause problems with stability of rabeprazole as it imbibes water into the formulation.

Another well known excipient for the stabilization of rabeprazole or its sodium salt is also magnesium oxide. Magnesium oxide is frequently used, since it also acts as an insoluble filler. It is very slightly soluble in pure water and the saturated solution thereof yields a pH of about 10. Its main disadvantage is that it is also very hygroscopic which is not beneficial in rabeprazole tablet production since rabeprazole is extremely sensitive to water.

As can be concluded from the above findings, a stable pharmaceutical composition of rabeprazole or a pharmaceutically acceptable salt thereof, such as rabeprazole sodium, is desired comprising non-hygroscopic excipients to prevent moistening and thus the decomposition of a rabeprazole salt.

### Summary of the invention

The inventors have developed a new and improved composition of rabeprazole sodium comprising calcium hydroxide as an effective and not hygroscopic alkalizing agent.

In the first aspect, the invention concerns a pharmaceutical composition comprising a tablet core comprising rabeprazole sodium, calcium hydroxide, at least one pharmaceutically acceptable excipient and a separating layer between the tablet core and the acidic enteric coating, wherein said composition comprises 0.1 to 30% of calcium hydroxide.

In another aspect, the invention concerns such pharmaceutical composition further comprising pharmaceutically acceptable excipients, such as non-alkaline fillers, diluents, binders, disintegrants, flow conditioners, anti-adherents, and lubricants.

In yet another aspect, the invention concerns such pharmaceutical composition wherein the separating layer comprises one of the excipients selected from the group of polyvinylpyrrolidone, hydroxypropylmethylcellulose (hypromellose) and a neutral polymethacrylate, such as poly(ethylacrylate, methylmethacrylate) 2:1.

In yet another aspect, the invention concerns the use of the composition according to one of the above cited aspects of the present invention for the preparation of a medicament for the prevention or treatment of peptic ulcers.

### Detailed description of the invention

Rabeprazole sodium is an active compound from a group of proton pump inhibitors, which are known to be unstable in neutral and acidic media.

For the preparation of the appropriate composition, an extensive investigation on compatibility of the rabeprazole sodium with various excipients carried out in order to select the optimal combination thereof in the final dosage form. The investigation of increase of related substances and degradation products for each of the selected excipients in the formulation after 7 days at 60 °C has been measured with HPLC. The results are presented in the Table 1:

**Table 1: Compatibility study results: increase in related substances and degradation products, after 7 days at 60 °C:**

| Ingredient | % Increase in related substances and degradation products |
|---|---|
| meglumine | 0.20 |
| sodium hydroxide | 0.45 |
| magnesium oxide | 0.27 |
| calcium hydroxide | 0.04 |
| L-arginine | 0.14 |
| anhydrous lactose | 12.06 |
| lactose monohydrate | 18.26 |
| sodium stearyl fumarate | 0.18 |

Surprisingly far the best results were achieved with calcium hydroxide.
A good stability of the formulation of the present invention lies the fact that calcium hydroxide is not hygroscopic. Therefore it does not adhere water and in such a manner rabeprazole sodium in the composition is further stabilized. Additionally an even distribution of calcium hydroxide assures that the tablet core of the pharmaceutical composition is evenly alkalized which contributes to the stability of the formulation of the present invention.

The solution of the problem of the present invention is therefore the preparation of a pharmaceutical formulation with rabeprazole sodium, calcium hydroxide as an alkalizing agent and compatible exipients. The pharmaceutical formulation of the present invention may be in the form of tablets having an enteric coating in order to protect the agent from the acidic gastric environment. After passing the tablet through the duodenum and jejunum, the enteric coating is dissolved and the active agent begins to dissolve from the formulation.

Additionally in the pharmaceutical formulation of the present invention, a separating layer between the tablet core, comprising rabeprazole sodium, and the acidic enteric coating of the tablet is applied in order to prevent interactions between the acid-sensitive abeprazole sodium and said acidic enteric coating. Thereby rabeprazole sodium remains stable within the tablet core.

Higher pH values in the tablet core obtained by the addition of calcium hydroxide provide a better stabilization of rabeprazole sodium whereas the partial solubility of calcium hydroxide ensures a better distribution of the alkalizing agent over the components of the tablet core. An additonal advantage of calcium hydroxyde is that it is safe to handle with, particularly as a water dispersion. The pharmaceutical composition allows a technically feasible process of designing a formulation with suitable biopharmaceutical properties.

The pharmaceutical composition of the present invention combines rabeprazole sodium, calcium hydroxide and non-alkaline excipients compatible with rabeprazole sodium. Said non-alkaline excipients may be selected from a group of mannitol, low substituted hydroxypropylcellulose, sodium stearyl fumarate and magnesium stearate.

The pharmaceutical composition of the present invention can also comprise one or more of other excipients, such as diluents, binders, disintegrants, flow conditioners, anti-adherents or lubricants.

Rabeprazole sodium can be typically present in the pharmaceutical composition of the present invention in an amount within the range of 5 to 50 %, preferably 7 to 25 %, more preferably 10 to 15 %, most preferably 11 to 13 %.

Calcium hydroxide is present in the pharmaceutical composition of the present invention in an amount within the range of 0.1 to 30 %, preferably from 0.5 % to 3 %, more preferably from 1.5 % to 3 %, most preferably 2 to 2.5 %.

For the pharmaceutical composition of the present invention, a separating layer is applied to prevent interactions between rabeprazole or the sodium salt thereof in the core and the acidic enteric coating. This separating layer can consist of one or more of the excipients, selected from the group of polyvinylpyrrolidone, hydroxypropylmethycellulose and neutral polymethacrylate (poly(ethyl acrylate, methyl methacrylate), 2 : 1).

The pharmaceutical composition of the present invention may be prepared by using standard techniques and manufacturing processes generally known in the art.
Tablet cores, for example, may be prepared by wet granulation. The components excluding the alkalizing agent and the lubricant may be blended, granulated with water or water/ethanol mixture with alkalizing agent suspended or dissolved in it. The obtained wet granulate is dried and passed through a mesh screen to obtain uniform particle size of the granules. A lubricant is added to the dry granulate and blending is continued until a lubricated granulate is obtained. The mixture is then compressed into tablets. Alternatively a direct compression technique known from the art can be employed.

Onto the tablet cores, a separating layer may be applied by using spray-coating of a water and/or ethanol-based film coating formulation. Coating ingredient combinations may be readily available and are usually fairly simple solutions of polymer in coating media.

Finally an enteric coating is applied by using spray-coating techniques with water or organic-based coating dispersions.

The composition of the present invention may be illustrated by the following examples, which in no way limit the scope of the invention.

### EXAMPLE 1

The ingredients of the composition of the present invention are presented in the table 2 below:

**Table 2:**

| | Ingredient | Mass percentage |
|---|---|---|
| Tablet core | | |
| | rabeprazole sodium | 11.5 |
| | calcium hydroxide | 0.6 |
| | mannitol | 56.1 |
| | low substituted hydroxypropylcellulose | 17.2 |
| | sodium stearyl fumarate | 0.9 |

| Separating layer | | |
|---|---|---|
| | polyvinylpyrrolidone | 1.7 |

| Enteric coating | | |
|---|---|---|
| | hydroxypropylmethylcellulose phthalate | 10.3 |
| | dibutylsebacate | 1.1 |
| | ferric oxide, yellow | 0.1 |
| | titanium dioxide | 0.5 |

The technological procedure to obtain the pharmaceutical composition of the present invention is the following:
Rabeprazole sodium, mannitol and low substituted hydroxypropylcellulose are mixed and granulated with water dispersion of calcium hydroxide in a high shear granulator. The obtained wet granulate is dried and passed through a mesh screen to obtain uniform particle size of the granules. Sodium stearyl fumarate is added to the dry granulate and mixed. The mixture is then compressed into tablets by using a rotary tablet machine. The tablet weight is 150 mg. Polyvinylpyrrolidone in dissolved in ethanol and applied onto tablet cores by using spray-coating in perforated drums.
For the enteric coating, hydroxypropylmethylcellulose phthalate, dibutylsebacate and pigments are dissolved/dispersed in ethanol-acetone (1:1) mixture. The obtained coating mixture is applied onto the tablet by using spray-coating in perforated drums.

### EXAMPLE 2

The ingredients of the composition of the present invention are presented in the table 3 below:

**Table 3:**

| | Ingredient | Mass percentage |
|---|---|---|
| Tablet core | | |
| | rabeprazole sodium | 11.5 |
| | calcium hydroxide | 3.0 |
| | mannitol | 45.1 |
| | low substituted hydroxypropyl cellulose | 25.8 |
| | magnesium stearate | 0.9 |

| Separating layer | | |
|---|---|---|
| | hydroxypropylmethylcellulose | 1.4 |
| | talc | 0.3 |

| Enteric coating | | |
|---|---|---|
| | hydroxypropylmethylcellulose phthalate | 10.3 |
| | dibutylsebacate | 1.1 |
| | ferric oxide, yellow | 0.1 |
| | titanium dioxide | 0.5 |

The technological procedure to obtain the pharmaceutical composition of the present invention is the following:
Rabeprazole sodium, mannitol, 70% calcium hydroxide and low substituted hydroxypropylcellulose are mixed and granulated with water dispersion of remaining 30 % calcium hydroxide in a high shear granulator. The obtained wet granulate is dried and passed through a mesh screen to obtain uniform particle size of the granules. Magnesium stearate is added to the dry granulate and mixed.
The mixture is then compressed into tablets by using a rotary tablet machine. The tablet weight is 150 mg. Hydroxypropyl-methylcellulose is dissolved and talc is dispersed in water/ethanol mixture (1:9). The obtained dispersion is applied onto the tablet cores by using spray-coating in perforated drums.
For the enteric coating, hydroxypropylmethylcellulose phthalate, dibutylsebacate and pigments are dissolved / dispersed in ethanol-acetone (1:1) mixture. Obtained coating mixture is applied onto the tablet by using spray coating in perforated drums.

### EXAMPLE 3

The ingredients of the composition of the present invention are presented in the table 4 below:

**Table 4:**

| | Ingredient | Mass percentage |
|---|---|---|
| Tablet core | | |
| | rabeprazole sodium | 12.0 |
| | calcium hydroxide | 2.1 |
| | mannitol | 55.3 |
| | low substituted hydroxypropyl cellulose | 18.0 |
| | sodium stearyl fumarate | 2.7 |

| Separating layer Separating layer | | |
|---|---|---|
| | hydroxypropylmethylcellulose | 2.0 |
| | talc | 0.4 |

| Enteric coating | | |
|---|---|---|
| | hydroxypropylmethylcellulose phthalate | 6.4 |
| | dibutylsebacate | 0.7 |
| | ferric oxide, yellow | 0.1 |
| | titanium dioxide | 0.3 |

The technological procedure to obtain the pharmaceutical composition of Example 3 is analogous to the one of Example 2 whereby sodium stearyl fumarate is used instead of magnesium stearate.

## Claims

1. A pharmaceutical composition comprising a tablet core comprising rabeprazole sodium, calcium hydroxide, at least one pharmaceutically acceptable excipient, and a separating layer between the tablet core and an acidic enteric coating of a tablet, wherein said composition comprises 0.1 to 30 % of calcium hydroxide.

2. The pharmaceutical composition according to claim 1 wherein pharmaceutically acceptable excipient is selected from the group of non-alkaline fillers, diluents, binders, disintegrants, flow conditioners, anti-adherents and lubricants.

3. The pharmaceutical composition according to claim 2 wherein non-alkaline excipients are selected from a group of mannitol, low substituted hydroxypropyl cellulose, sodium stearyl fumarate and magnesium stearate.

4. The pharmaceutical composition according to claim 1 wherein the separating layer comprises at least one of the excipients selected from the group of polyvinylpyrrolidone, hydroxypropylmethylcellulose and neutral polymethacrylate.

5. The pharmaceutical composition according to claim 1 wherein said composition comprises 5 to 50 % of rabeprazole sodium.

6. The pharmaceutical composition according to claim 5 wherein said composition comprises about 7 to 25 % of rabeprazole sodium.

7. The pharmaceutical composition according to claim 1 wherein said composition comprises about 0.5 to 3 % of calcium hydroxide.

8. The pharmaceutical composition according to claim 7, wherein said composition comprises about 1.5 to 3 % of calcium hydroxide.

9. The use of the composition according to any of claims 1 to 8 for the preparation of a medicament for prevention or treatment of peptic ulcers.

## Patentansprüche

1. Pharmazeutische Zusammensetzung, die einen Tablettenkern, der Rabeprazol-Natrium, Calciumhydroxid, mindestens einen pharmazeutisch geeigneten Hilfsstoff umfasst, und eine Trennschicht zwischen dem Tablettenkern und einem sauren enterischen Überzug einer Tablette umfasst, wobei die Zusammensetzung 0,1 bis 30% Calciumhydroxid umfasst.

2. Pharmazeutische Zusammensetzung gemäß Anspruch 1, wobei der pharmazeutisch geeignete Hilfsstoff ausgewählt ist aus der Gruppe von nicht-alkalischen Füllmitteln, Verdünnungsmitteln, Bindemitteln, Sprengmitteln, Fließverbesserern, Antihaftmitteln und Gleitmitteln.

3. Pharmazeutische Zusammensetzung gemäß Anspruch 2, wobei nicht-alkalische Hilfsstoffe ausgewählt werden aus der Gruppe von Mannitol, niedrig substituierter Hydroxypropylcellulose, Natriumstearylfumarat und Magnesiumstearat.

4. Pharmazeutische Zusammensetzung gemäß Anspruch 1, wobei die Trennschicht mindestens einen der Hilfsstoffe ausgewählt aus der Gruppe von Polyvinylpyrrolidon, Hydroxypropylmethylcellulose und neutralem Polymethacrylat, umfasst.

5. Pharmazeutische Zusammensetzung gemäß Anspruch 1, wobei die Zusammensetzung 5 bis 50% Rabeprazol-Natrium umfasst.

6. Pharmazeutische Zusammensetzung gemäß Anspruch 5, wobei die Zusammensetzung etwa 7 bis 25% Rabeprazol-Natrium umfasst.

7. Pharmazeutische Zusammensetzung gemäß Anspruch 1, wobei die Zusammensetzung etwa 0,5 bis 3% Calciumhydoxid umfasst.

8. Pharmazeutische Zusammensetzung gemäß Anspruch 7, wobei die Zusammensetzung etwa 1,5 bis 3% Calciumhydroxid umfasst.

9. Verwendung der Zusammensetzung gemäß irgendeinem der Ansprüche 1 bis 8 für die Herstellung eines Medikaments zur Vorbeugung oder Behandlung von Magengeschwüren.

## Revendications

1. Une composition pharmaceutique comprenant un noyau de comprimé comprenant du sodium de rabéprazole, d'hydroxyde de calcium, au moins un excipient acceptable du point de vue pharmaceutique, et une couche de séparation entre le noyau de comprimé et une enduction entérique acide d'un comprimé, où ladite composition comprend entre 0,1 et 30 % d'hydroxyde de calcium.

2. La composition pharmaceutique selon la revendication 1 où l'excipient acceptable du point de vue pharmaceutique est sélectionné parmi le groupe comportant des remplissages, des diluants, des liants, des délitants, des conditionneurs de flux, des anti-adhérents et des lubrifiants non alcalins.

3. La composition pharmaceutique selon la revendication 2 où l'excipient non alcalin parmi le groupe comportant le mannitole, de la cellulose de hydroxypropyle faiblement substitué, de fumarate de stéaryle de sodium et de stéarate de magnésium.

4. La composition pharmaceutique selon la revendication 1 où la couche de séparation comprend au moins un des excipients sélectionné parmi le groupe comportant le polyvinylpyrrolidone, la cellulose de hydroxypropylméthyle et le polymethacrylate neutre.

5. La composition pharmaceutique selon la revendication 1 où ladite composition comprend entre 5 et 50 % de sodium de rabéprazole.

6. La composition pharmaceutique selon la revendication 5 où ladite composition comprend entre 7 et 25 % de sodium de rabéprazole.

7. La composition pharmaceutique selon la revendication 1 où ladite composition comprend environ 0,5 à 3 % d'hydroxyde de calcium.

8. La composition pharmaceutique selon la revendication 7 où ladite composition comprend environ 1,5 à 3 % d'hydroxyde de calcium.

9. L'utilisation de la composition selon n'importe laquelle des revendications 1 à 8 pour la préparation d'un médicament pour
